**Europäisches Patentamt**

**European Patent Office**

**Office européen des brevets**

(19)

(11) Veröffentlichungsnummer : **0 055 796**
**B1**

(12) **EUROPÄISCHE PATENTSCHRIFT**

(45) Veröffentlichungstag der Patentschrift :
28.11.84

(21) Anmeldenummer : **81107971.4**

(22) Anmeldetag : **06.10.81**

(51) Int. Cl.³ : **C 07 C 89/02, C 07 C 91/26**

(54) **Verfahren zur Herstellung von wasserfreiem 2-Hydroxy-3-chlorpropyltrialkylammoniumchlorid.**

(30) Priorität : **31.12.80 NL 8007126**

(43) Veröffentlichungstag der Anmeldung :
**14.07.82 Patentblatt 82/28**

(45) Bekanntmachung des Hinweises auf die Patenterteilung : **28.11.84 Patentblatt 84/48**

(84) Benannte Vertragsstaaten :
**AT BE CH DE FR GB IT LI NL SE**

(56) Entgegenhaltungen :
**DE-A- 2 156 569**

(73) Patentinhaber : **Chemische Fabriek Zaltbommel B.V.**
**Postbus 52 Koxkampseweg 14-20**
**Zaltbommel (NL)**

(72) Erfinder : **Van der Maas, Hendrikus Joh. H.**
**De Boogerd 25**
**NL-5305 CK Zullichem Brakel (NL)**

(74) Vertreter : **Siecke, Wolf-Friedrich, Dr.**
**c/o DYNAMIT NOBEL AKTIENGESELLSCHAFT HA**
**Patente/Patentabteilung Postfach 1209 Kölner**
**Strasse**
**D-5210 Troisdorf/Bez. Köln (DE)**

## Beschreibung

Gegenstand der vorliegenden Erfindung ist ein Verfahren zur Herstellung von wasserfreiem 2-Hydroxi-3-chlorpropyltrialkylammoniumchlorid, bei dem Trialkylammoniumchlorid mit Epichlorhydrin umsetzt wird.

Die Umsetzung von Epichlorhydrin mit Trimethylammoniumchlorid unter Bildung von 2-Hydroxi-3-chlorpropyltrimethylammoniumchlorid ist z. B aus der US-PS 28 76 217 bekannt. Bei diesem Verfahren wird das Trimethylammoniumchlorid als wässrige Lösung eingesetzt. Es entsteht deshalb als Reaktionsprodukt das 2-Hydroxi-3-chlorpropyltrimethylammoniumchlorid in Form einer wässrigen Lösung. Für die Weiterverwendung dieses Produktes zur Herstellung von kationenaktiven Stärkeprodukten wird im allgemeinen diese wässrige Lösung nach ihrer Reinigung eingesetzt.

Nachteilig bei der in der US-PS 28 76 217 beschriebenen Verfahrensweise ist das Arbeiten im wässrigen Milieu, das zu einer Reihe von Nebenreaktionen führt, wie z. B. die Bildung von 1,3-Bistrimethylammonium-2-hydroxipropan-di-chlorid. Dieses Nebenprodukt fällt bei dieser Herstellung mit an und wird von dem gewünschten Endprodukt in den meisten Fällen nicht abgetrennt. Da es jedoch mit Stärke nicht reagiert, ist seine Anwesenheit in dem gewünschten Endprodukt unerwünscht.

Weiterhin entsteht bei der Verfahrensweise der US-PS 28 76 217 durch Reaktion des Epichlorhydrins mit Salzsäure 1,3-Dichlorpropanol-2, welches mit Stärke unter Vernetzung reagiert. Der Gehalt an diesem Propanol muß in dem gewünschten Endprodukt deshalb unter 50 ppm liegen.

Es wurde deshalb auch bereits vorgeschlagen, die Herstellung des 2-Hydroxi-3-chlorpropyltrimethylammoniumchlorids in einem wasserfreien organischen Lösungsmittel durchzuführen, um zu einem wasserfreien Produkt zu gelangen, das man bei der Herstellung als Niederschlag abfiltrieren kann und das dann in kristalliner Form vorliegt (vgl. NOA 78 04 880). Bei diesem Verfahren wird jedoch von gasförmigem Trimethylamin ausgegangen, das mit dem eingesetzten Lösungsmittel zuerst reagiert. Wegen der Gesundheitsgefährdung des dabei eingesetzten Lösungsmittels ist die technische Durchführung des Verfahrens mit zusätzlichen Problemen hinsichtlich der Reinheit der Arbeitsluft belastet.

Auch bei dem in der DE-OS 21 56 569 beschriebenen Verfahren zur Herstellung von 2-Hydroxy-3-chlorpropyltrialkylammoniumchloriden wird als einer der Reaktionspartner ein Trialkylamin eingesetzt. Dabei muß jedoch eine der Alkylgruppen eine langkettige Alkylgruppe sein, so daß Verbindungen entstehen, bei denen am N-Atom mindestens ein langkettiger Alkylrest steht. Als weiterer Reaktionspartner wird Dichlorpropanol im Überschuß eingesetzt. Bei der Übertragung dieser Verfahrensweise auf die Herstellung der gewünschten 2-Hydroxy-3-chlorpropylammoniumchloride erhält man jedoch nicht Produkte der oben genannten gewünschten Reinheit.

Es bestand deshalb die Aufgabe, 2-Hydroxy-3-chlorpropyltrialkylammoniumchlorid in wasserfreier Form herzustellen, wobei als Ausgangsprodukt eine wässrige Lösung von Trialkylammoniumchlorid eingesetzt werden soll. Weiterhin bestand die Aufgabe, das wasserfreie Produkt in möglichst großer Reinheit und mit hohen Ausbeuten zu erhalten. Nebenprodukte, wie das oben genannte 1,3-Bistrimethylammonium-2-hydroxipropanchlorid oder das 1,3-Dichlorpropanol-2 sollen nach Möglichkeit in dem Endprodukt nicht vorhanden sein.

In Erfüllung dieser Aufgabe wurde nun ein Verfahren zur Herstellung von wasserfreiem 2-Hydroxi-3-chlorpropyltrialkylammoniumdichlorid durch Umsetzen von Trialkylammoniumchlorid mit Epichlorhydrin gefunden, das dadurch gekennzeichnet ist, daß man ein wässrige Lösung von Trialkylammoniumchlorid in einem organischen Lösungsmittel, das mit Wasser ein bei Temperaturen unterhalb seines Siedepunkts siedendes Azeotrop bildet, auf Temperaturen bis zum Siedepunkt dieses Lösungsmittels erhitzt und das sich bildende Azeotrop abdestilliert und anschließend in dem Lösungsmittel die Umsetzung mit Epichlorhydrin bei Temperaturen zwischen 20 und 120 °C durchführt.

Bei der Durchführung dieser Verfahrensweise erhält man das gewünschte Endprodukt direkt in fester Form und in Ausbeuten über 80 %. Die Reinheit des Produktes, gemessen an dessem Chlorhydringehalt und durch NMR-Analyse, beträgt bis zu 99,8 %. Das Produkt ist demzufolge bei den bevorzugten Durchführungsformen des Verfahrens praktisch frei von 1,3-Bistrialkylammonium-2-hydroxipropandichlorid.

Wichtig bei der Durchführung des erfindungsgemäßen Verfahrens ist die Auswahl der einzusetzenden Lösungsmittel. Diese Lösungsmittel müssen ein Azeotrop mit Wasser bilden, wobei dieses Azeotrop einen Siedepunkt haben soll, der unterhalb des Siedepunkts des wasserfreien Lösungsmittels liegt und wobei das Azeotrop sich vorzugsweise leicht in zwei Schichten trennen soll. Außerdem muß das gewählte Lösungsmittel oder Lösungsmittel-Gemisch keine Reaktion mit Epichlorhydrin unter den gewählten Reaktionsbedingungen zeigen. Diese Bedingung erfüllen im allgemeinen solche organischen Lösungsmittel, die nur ein eingeschränktes Lösungsvermögen für Wasser besitzen. Zu diesen Lösungsmitteln zählen aliphatische Alkohole mit 4 bis 6 C-Atomen, aliphatische gesättigte Kohlenwasserstoffe mit 5 bis 8 C-Atomen, aromatische Kohlenwasserstoffe wie Toluol Benzol, sowie Trichloräthylen, Perchloräthylen und Methyläthylketon. Auch Gemische dieser Verbindungen können eingesetzt werden. In einigen Fällen zeigen Gemische aus Lösungsmitteln noch bessere Wirkungen als die Lösungsmittel selber, so daß der Einsatz solcher Gemische eine bevorzugte Durchführungsform des beanspruchten Verfahrens sind.

Zu den bevorzugt einsetzbaren Gemischen zählen solche aus den genannten aliphatischen Alkoholen mit Toluol oder Benzol, wobei das Verhältnis der beiden Komponenten bevorzugt so gewählt werden soll, daß das azeotrope Gemisch dieser beiden Lösungsmittel eingesetzt wird. Diese Bedingung gilt auch für andere Gemische aus den einsetzbaren Lösungsmitteln.

Im ersten Schritt des erfindungsgemäßen Verfahrens wird eine wasserhaltige Lösung eines Trialkylammoniumchlorids in dem Lösungsmittel bzw. dem Lösungsmittelgemisch aufgelöst bzw. dispergiert. Im allgemeinen wird eine 60 bis 75 %ige wässrige Lösung eingesetzt ; jedoch können auch wässrige Lösungen geringerer oder höherer Konzentration in gleicher Weise verarbeitet werden. Es ist jedoch günstig, eine nicht zu verdünnte Lösung des Trialkylammoniumchlorids einzusetzen, um nicht zuviel Wasser abdestillieren zu müssen.

Die Menge der einzusetzenden Lösung des Trialkylammoniumchlorids hängt von dem Lösungsvermögen des Lösungsmittels für dieses Salz ab. Im allgemeinen werden etwa 5 bis 8 Mol Trialkylaminhydrochlorid pro 2 000 g Lösungsmittel oder Lösungsmittel-Gemisch eingesetzt. Geringere Konzentrationen an Trialkylaminhydrochlorid in dem gewählten Medium können selbstverständlich auch eingesetzt werden ; sie bringen jedoch keine Vorteile.

Die Lösung des Trialkylaminhydrochlorids in dem organischen Lösungsmittel wird anschließend auf die Temperatur des Siedepunkts des organischen Lösungsmittels erhitzt und das sich bildende azeotrope Gemisch unter Rückfluß abdestilliert. Das in dem abdestillierten Azeotrop anfallende Wasser, wird auf an sich bekannte Weise (z. B. durch Dekantieren) abgetrennt, während das dabei zurückgewonnene Lösungsmittel nach Möglichkeit wieder das Reaktionsgemisch zurückgeführt wird. Gegebenenfalls kann fraktioniert destilliert werden, damit nur das azeotrope Gemisch überdestilliert. Das Erhitzen wird so lange fortgesetzt, bis im Destillat die der eingesetzten wässrigen Lösung des Trialkylaminhydrochlorids entsprechende Menge Wasser angefallen ist. Diese Zeit läßt sich in Vorversuchen leicht ermitteln ; sie ist von mehreren an sich bekannten Faktoren abhängig.

Während des azeotropen Abdestillierens des Wassers soll sich das Trialkylammoniumchlorid teilweise oder vollständig als fester Stoff abscheiden ; letzteres hängt von dem gewählten Reaktionsmedium ab. Ein solches Ausfallen stört den weiteren Verlauf der Reaktion nicht. Es muß nur dafür gesorgt werden, daß die erhaltene Dispersion noch gut rührbar ist. Gegebenenfalls muß noch wasserfreies Lösungsmittel oder Lösungsmittelgemisch hinzugefügt werden. Nach dem azeotropen Abdestillieren des Wassers kann dabei auch ein beliebiges Lösungsmittel, wie z. B.

ein niedriger, mit Wasser mischbarer Alkohol, hinzugefügt werden.

Nach dem Abdestillieren des Wassers wird der Lösung oder Suspension Epichlorhydrin hinzugefügt. Es werden bevorzugt äquimolekulare Mengen an Epichlorhydrin eingesetzt. Die Reaktion mit dem Trialkylammoniumchlorid läuft bereits bei leicht erhöhten Temperaturen ab. Der bevorzugte Temperaturbereich liegt zwischen 40 und 60 °C ; da die Umsetzung exotherm ist, muß gegebenenfalls gekühlt werden. Der Verlauf der Reaktion läßt sich gaschromatographisch durch Bestimmung des Epichlorhydringehalts der Lösung verfolgen. Das gewünschte Endprodukt fällt als weißes kristallines Pulver in dem Lösungsmittel oder dem Lösungsmittelgemisch an. Es kann nach beendeter Reaktion direkt abfiltriert werden und wird auf an sich bekannte Weise aufgearbeitet, wobei man die oben erwähnte Reinheit direkt erhält.

Das bevorzugt eingesetzte Trialkylammoniumchlorid ist das Trimethylammoniumchlorid. Weitere mögliche Alkylgruppen sind solche mit 2 bis 4 C-Atomen. Die Alkylgruppen können auch unterschiedlich sein.

Beispiel 1

In einen 3 l Rundkolben, versehen mit Rührer, Thermometer, Tropftrichter und einer Destillierbrücke mit Wasserabscheider und aufgesetztem Rückflußkühler werden 2 100 ml Toluol eingefüllt. Anschließend werden 680 g einer 70 %igen wässrigen Trimethylammoniumchlorid-Lösung hinzugefügt. Der theoretische Gehalt an Trimethylammoniumchlorid betrug 5 Mol.

Das Gemisch wird auf Siedetemperatur erhitzt und im Laufe von sechs Stunden war das Wasser azeotropisch abdestilliert und abgetrennt. Es werden insgesamt 201 ml Wasser aufgefangen. In die verbleibende, toluolhaltige Kristallmasse werden im Verlauf von 1 1/2 Stunden insgesamt 462,5 g Epichlorhydrin bei einer Anfangstemperatur von 70 bis 75 °C hinzugefügt. Die Innentemperatur stieg im Laufe der Zeit auf 98 bis 100 °C an. Nach beendeter Zuführung ließ man noch insgesamt sechs Stunden nachrühren, wobei das Reaktionsgemisch langsam abkühlte.

Der entstehende Feststoff wurde abfiltriert, mit Toluol und anschließend mit Aceton nachgewaschen. Anschließend wurde der Feststoff im Vakuum getrocknet. Es wurden 812 g weißes Pulver erhalten. Die Ausbeute betrug demzufolge 83,7 %, berechnet auf Trimethylaminhydrochlorid.

Die Reinheit des erhaltenen Produkts lag zwischen 96 und 98 %.

Beispiel 2

In der in Beispiel 1 genannten Apparatur werden 2 000 ml Trichloräthylen vorgelegt und 543 g einer 70 %ig wässrigen Lösung von Trimethylammoniumchlorid hinzugefügt. Das Gemisch wird zum Sieden erhitzt und das azeotrope Gemisch von Trichloräthylen/Wasser wird im Laufe von 11 Stunden abdestilliert. Es werden insgesamt 159,5 ml Wasser aufgefangen.

Anschließend wurde das erhaltene Gemisch auf 75 °C abgekühlt und bei dieser Temperatur mit dem Zufügen von Epichlorhydrin begonnen. Insgesamt wurden 370,1 g Epichlorhydrin (4 Mol) im Laufe von 3 Stunden eintropfen gelassen. Dabei stieg die Innentemperatur des Gemischs auf 90 °C an. Es wird noch zwei Stunden nachgerührt, bis kein Epichlorhydrin gaschromatographisch mehr nachgewiesen werden konnte.

Anschließend wurde der erhaltene Feststoff abfiltriert, mit Trichloräthylen gewaschen und getrocknet. Es wurden insgesamt 672 g mit einem Aktivgehalt von 93,4 % erhalten. Die Ausbeute betrug demzufolge 83,5 %, bezogen auf Trimethylammoniumchlorid.

Beispiel 3

In gleicher Weise wie im Beispiel 1 wurden 2 000 ml Methylisobutylketon mit 545,7 g einer 70 %igen wässrigen Lösung von Trimethylammoniumchlorid versetzt und das Wasser als azeotropes Gemisch abdestilliert. Es wurden im Verlaufe von 7 Stunden 160,4 ml Wasser erhalten.

Anschließend wird auf 80 °C abgekühlt und im Laufe von 5 Stunden 370,7 g Epichlorhydrin hinzugefügt. Während des Zufügens stieg die Temperatur auf 90 bis 95 °C an.

Ohne Nachrühren wird das erhaltene Produkt sofort abfiltriert und mit Methylisobutylketon gewäschen. Das Trocknen erfolgte wiederum im Vakuum.

Es wurden insgesamt 577 g 2-Hydroxi-3-chlorpropyltrimethylammoniumchlorid mit einer Reinheit von 97 bis 98 % erhalten. Die Ausbeute lag bei 75,1 %.

Beispiel 4

In dem in Beispiel 1 genannten Dreihalskolben werden 2 000 ml n-Butanol vorgelegt und auf 117 °C Innentemperatur unter Rückfluß erhitzt. Durch einen Tropftrichter werden im Laufe von 4 Stunden 1 090 g einer 70 %igen wässrigen Lösung von Trimethylaminhydrochlorid hinzugefügt ; dabei fiel die Innentemperatur auf 95 bis 98 °C ab. Bei dieser Temperatur wurde das Gemisch während 8 Stunden unter Rückfluß erhitzt, wobei insgesamt 306 g Wasser plus Butanol im Wasserabscheider aufgefangen wurden. Der Wassergehalt dieses Gemischs betrug 285 ml.

Das Reaktionsgemisch wurde daraufhin auf 60 bis 70 °C abgekühlt und im Laufe von 3 Stunden 740 g Epichlorhydrin hinzugefügt. Dabei stieg die Temperatur so stark an, daß das Gemisch gekühlt werden mußte. Nach Beendigung des Zusatzes wurde während 5 Stunden nachgerührt und anschließend der erhaltene Feststoff abfiltriert, mit Butanol gewaschen und daraufhin getrocknet. Es wurden 1 373 g 2-Hydroxi-3-chlorpropyltrimethylammoniumchlorid mit einer Reinheit von 88 bis 93 % erhalten.

Beispiel 5

In einem 3 l Dreihalskolben wird ein Lösungsmittelgemisch aus 540 g n-Butanol und 1 460 g Toluol vorgelegt. Dieses Gemisch hat einen Siedepunkt von 105,6 °C. Es wurde unter Rückfluß auf Siedetemperatur erhitzt. Durch einen Tropftrichter wurden 1 090 g einer 70 %igen wässrigen Lösung von Trimethylaminhydrochlorid zutropfen gelassen. Dabei fiel die Innentemperatur auf 100 °C ab. Im Verlauf von 7 Stunden unter Rückfluß und Auffangen des wasserhaltigen azeotropen Gemischs wurden 335 g Wasserdestillat mit einem Gehalt von 97,8 % Wasser aufgefangen.

Die erhaltene Kristallmasse von Trimethylammoniumchlorid in Toluol/Butanol wurde anschließend auf 50 °C abgekühlt und im Verlaufe von 3 1/2 Stunden wurden diesem Gemisch 740 g Epichlorhydrin hinzugefügt. Durch Kühlung wurde die Temperatur auf maximal 70 °C gehalten. Nach Beendigung des Zulaufs von Epichlorhydrin wurde noch 4 Stunden nachgerührt, bis kein Epichlorhydrin mehr gaschromatographisch nachgewiesen werden konnte.

Anschließend wurde das Reaktionsgemisch abgekühlt, der Feststoff abfiltriert und mit Toluol nachgewaschen und anschließend getrocknet. Es wurden 1 299 g eines weißen kristallinen Pulvers erhalten. Sein aktiver Gehalt an 2-Hydroxi-3-chlorpropyltrimethylammoniumchlorid lag zwischen 99,6 und 99,7 %. Es konnten nur 24 ppm Dichlorpropanol in dem Produkt nachgewiesen werden.

**Ansprüche**

1. Verfahren zur Herstellung von wasserfreiem 2-Hydroxi-3-chlorpropyltrialkylammoniumchlorid durch Umsetzung von Trialkylammoniumchlorid mit Epichlorhydrin, dadurch gekennzeichnet, daß man eine wässrige Lösung eines Trialkylammoniumchlorids in einem organischen Lösungsmittel, das mit Wasser ein bei Temperaturen unterhalb seines Siedepunkts siedendes Azeotrop bildet, auf Temperaturen bis zum Siedepunkt dieses Lösungsmittels erhitzt, das sich bildende Azeotrop abdestilliert und anschließend in dem Lösungsmittel die Umsetzung mit Epichlorhydrin bei Temperaturen zwischen 20 und 120 °C durchführt.

2. Verfahren gemäß Anspruch 1, dadurch gekennzeichnet, daß man die Umsetzung bei Temperaturen zwischen 40 und 60 °C durchführt.

3. Verfahren gemäß Ansprüchen 1 oder 2, dadurch gekennzeichnet, daß die einzelnen Verfahrensschritte teilweise oder vollständing in einem Gemisch der organischen Lösungsmittel durchgeführt werden.

**Claims**

1. Process for the production of water-free 2-hydroxy-3-chloropropyltrialkylammonium chloride by reaction of trialkylammonium chloride with epichlorohydrin, characterised in that an aqueous solution of a trialkylammonium chloride in an organic solvent which forms a boiling azeotrope with water at temperatures below its boiling point, is heated to temperatures up to the boiling point of this solvent, the azeotrope being formed is distilled off and finally the reaction with epichlorohydrin is carried out in the solvent at temperatures between 20 and 120 °C.

2. Process according to claim 1, characterised in that the reaction is carried out at temperatures between 40 and 60 °C.

3. Process according to claims 1 or 2, characterised in that the individual process steps are partially or completely carried out in a mixture of the organic solvents.

**Revendications**

1. Procédé de préparation d'un chlorure de 2-hydroxy-3-chloropropyl-trialkylammonium anhydre par réaction d'un chlorure de trialkylammonium avec l'épichlorhydrine, caractérisé en ce qu'on chauffe une solution aqueuse d'un chlorure de trialkylammonium dans un solvant organique formant, avec l'eau, un azéotrope bouillant à des températures inférieures à son point d'ébullition, à des températures allant jusqu'au point d'ébullition de ce solvant, on sépare l'azéotrope qui se forme, par distillation, puis on effectue la réaction avec l'épichlorhydrine dans le solvant à des températures se situant entre 20 et 120 °C.

2. Procédé suivant la revendication 1, caractérisé en ce qu'on effectue la réaction à des températures comprises entre 40 et 60 °C.

3. Procédé suivant la revendication 1 ou 2, caractérisé en ce qu'on effectue les étapes opératoires individuelles partiellement ou complètement dans un mélange des solvants organiques.